Europäisches Patentamt

European Patent Office

Office européen des brevets

(11) Numéro de publication: **0 299 878**
**A1**

## DEMANDE DE BREVET EUROPEEN

(21) Numéro de dépôt: **88401848.2**

(22) Date de dépôt: **15.07.88**

(51) Int. Cl.⁴: **C 12 Q 1/28**
**G 01 N 33/535**

(30) Priorité: **16.07.87 FR 8710050**

(43) Date de publication de la demande:
**18.01.89 Bulletin 89/03**

(84) Etats contractants désignés:
**AT BE CH DE ES FR GB GR IT LI LU NL SE**

(71) Demandeur: **IRE-CELLTARG S.A.**
**Zone Industrielle**
**B-6220 Fleurus (BE)**

(72) Inventeur: **Abarca, Jorge**
**Av. H. Matisse 11 Bte 14**
**B-1140 Bruxelles (BE)**

(74) Mandataire: **Warcoin, Jacques et al**
**Cabinet Régimbeau 26, avenue Kléber**
**F-75116 Paris (FR)**

(54) **Technique enzymatique utilisant un chromogène lyophilise, trousse de réactif et procédé de lyophilisation.**

(57) L'invention concerne une technique enzymatique utilisant la révélation de la peroxydase avec comme chromogène le DAB (tétrahydrochlorure de 3,3' diaminobenzidine), caractérisée en ce qu'on fait usage du DAB conservé sous forme lyophilisée que l'on remet en solution en milieu aqueux.

La présente invention concerne également une trousse de réactifs pour la mise en oeuvre de cette technique ainsi qu'un procédé de lyophilisation du DAB.

EP 0 299 878 A1

**Description**

**TECHNIQUE ENZYMATIQUE UTILISANT UN CHROMOGENE LYOPHILISE, TROUSSE DE REACTIF ET PROCEDE DE LYOPHILISATION**

La présente invention concerne d'une manière générale les techniques enzymatiques utilisant la propriété de certaines enzymes simples comme la peroxydase de transformer un substrat incolore et soluble en un produit coloré, qui éventuellement précipite, permettant ainsi une visualisation rapide.

Plus particulièrement, la présente invention concerne les techniques immunohistochimiques mettant en oeuvre une réaction immunologique, par exemple les techniques de dosages immunologiques, utilisant un marquage enzymatique.

Pour ces marquages, des enzymes à haute activité ont déjà été proposées, telles que la peroxydase du raifort, la glucose oxydase d'origine fongique, la β galactosidase d'origine bactérienne.

Ces techniques requièrent l'utilisation de substrats spécifiques de ces enzymes qui donnent naissance, après action enzymatique, selon les cas :
- à des dérivés insolubles, colorés ou opaques aux faisceaux d'électrons. Ces substrats sont utilisés en microscopies optique et électronique ;
- à des dérivés colorés solubles. La densité optique peut être mesurée par les techniques photométriques courantes. D'une façon plus générale, toute action enzymatique donnant naissance à un phénomène mesurable peut être utilisée.

Plus précisément, la présente invention concerne les techniques enzymatiques utilisant la peroxydase, notamment la peroxydase du raifort.

La méthode la plus fréquemment utilisée fait appel à des anticorps spécifiques marqués à la peroxydase, ceux-ci sont révélés dans les coupes destinées à la microscopie optique par addition de dérivés de la benzidine, notamment le tétrahydrochlorure de 3,3′ diaminobenzidine qui, sous l'action de la peroxydase et en présence d'eau oxygénée, vont donner naissance à des dépôts bruns sombres. Cette méthode est utilisée par exemple dans les dosages non compétifis du type sandwich. Il suffit d'ajouter le révélateur chromogène et de stopper l'action enzymatique au moment où le contraste optimum est obtenu, c'est-à-dire de faire une mesure de la densité optique à la longueur d'onde optimale du chromogène. Plus la densité optique sera élevée, plus il y aura d'antigène.

Les anticorps marqués à la peroxydase sont également partiulièrement bien adaptés à l'utilisation en microscopie électronique. Le principe de ces techniques est le suivant : comme pour la technique précédente, le conjugué anticorps-peroxydase fixé sur les sites antigéniques est mis en présence de 3,3′ diaminobenzidine et d'eau oxygénée, mais aussi de tétroxyde d'osmium. Le polymère oxydé de diaminobenzidine qui se forme a la propriété de former des chélates insolubles avec l'oxyde d'osmium, qui sont très denses aux électrons. C'est ce chélate qui, après les lavages, usuels, est mis en évidence en microscopie électronique.

Dans la technologie des sondes nucléiques, la détection de ces sondes fait également appel à des systèmes enzymatiques tels que la phosphatase alcaline ou la peroxydase. De nombreuses techniques ont été développées. Elles sont fondées :
- soit sur la détection indirecte de l'hybride par des systèmes utilisant des molécules intermédiaires capables de reconnaître des motifs fixés sur la sonde nucléique. La reconnaissance est alors du type protéine-coenzyme ou anticorps-haptènes, ces molécules étant elles-mêmes couplées directement ou non à des enzymes de visualisation ;
- soit sur la détection directe de l'hybride par couplace covalent d'une enzyme à la sonde.

Il a par exemple été développé une procédure utilisant un analogue de base : le 5-bromodéoxyuridine (5-BdUr). Cet analogue est incorporé dans la sonde par la technique de déplacement de coupure (nick-translation). L'hybride est visualisé après reconnaissance 5-BdUr par un anticorps monoclonal de souris anti-5-BdUr. L'anticorps de souris est ensuite lui-même reconnu par un anticorps de mouton anti-souris couplé à la peroxydase et la visualisation proprement dite a lieu par transformation du substrat incolore en produit coloré par l'action de cette enzyme.

De nombreuses techniques ont également été développés pour coupler directement, de manière covalente, la peroxydase à des acides nucléiques. Le polyéthylèneimine par exemple a été utilisé pour coupler la peroxydase à l'ADN en présence de glutaraldéhyde.

La présente invention a donc pour objet une technique enzymatique utilisant la révélation de la peroxydase par un chromogène consistant dans le DAB (tétrahydrochlorure de 3,3′ diaminobenzidine).

Plus particulièrement, la présente invention a pour objet des technique immunologiques, notamment de dosages, mettant en oeuvre des anticorps marqués à la peroxydase et, comme révélateur chromogène, le tétrahydrochlorure de 3,3′ diaminobenzidine (DAB).

La présente invention a également pour objet les techniques enzymatiques mettant en oeuvre des sondes d'acides nucléiques et un marquage à la peroxydase.

La présente invention a donc naturellement pour objet une trousse de réactifs pour la mise en oeuvre d'une des ces techniques enzymatiques, notamment un "kit" de dosage et/ou diagnostique comportant du DAB.

La présente invention a donc également pour objet des "kits" ou trousses de réactifs pour techniques immunologiques, notamment des dosages, comportant des anticorps marqués à la peroxydase et comportant notamment, comme réactif chromogène, du tétrahydrochlorure de 3,3′ diaminobenzidine (DAB).

La présente invention a également pour objet un "kit" de dosage et/ou diagnostique comportant une sonde d'acide nucléique marquée à la peroxydase, ainsi que du DAB.

Ce révélateur chromogène est très efficace. Il constitue même un réactif idéal pour l'immunohistochimie impliquant les techniques immunoperoxydasiques, comme on l'a vu. Cependant, il présente l'inconvénient d'être très toxique sous forme de poudre. Le DAB est en effet considéré alors comme très hépatotoxique ou hépatocarcinogène. Les manipulations de ce produit peuvent donc entraîner des risques de contamination et sont à éviter au maximum. Mais ce produit, lorsqu'il est livré en poudre, même congelé et conservé à 4°C, se dégrade rapidement. C'est pourquoi il est actuellement proposé, notamment dans les trousses immunohistochimiques, sous forme de poudre. Conservé dans cette présentation, le DAB offre alors une stabilité suffisante, pourvu qu'il soit conservé à 4°C. Toutefois, son utilisation nécessite une étape de manipulation dangereuse pour le remettre en solution et surtout pour le filtrer. Ce qui est indispensable lorsqu'il est présenté initialement sous forme de poudre, compte tenu des difficultés de solubilisation existantes.

Le problème qui se pose avec le DAB n'a encore jamais été résolu et les kits actuellement proposés comportent du DAB très toxique présenté sous forme de poudre. Il a été envisagé de remplacer le DAB par une autre substance non toxique, mais les caractéristiques particulièrement avantageuses de celui-là n'ont jamais pu être retrouvées. Ainsi de nombreux kits utilisent le chromogène AEC, mais celui-ci donne un précipité moins important d'une part et d'autre part n'offre pas la possibilité de procéder à la déshydratation des lames contrairement au DAB. Il en résulte que les lames marquées au DAB se conservent plus longtemps.

Le but de la présente invention est donc de trouver une présentation pour la conservation du DAB qui évite les manipulations dangereuses du produit lors de son utilisation, tout en lui conférant une stabilité améliorée par rapport à sa conservation en solution.

On a découvert, selon la présente invention, qu'en conservant le DAB sous forme lyophilisée ces conditions étaient remplies, le produit étant en effet quasiment prêt à l'emploi sans manipulation dangereuse, notamment sans étape de filtration d'une part et conservant toutes ses propriétés chromogènes d'autre part.

Selon la caractéristique essentielle de la présente invention, le DAB est donc présenté et conservé sous forme lyophilisée, puis remis en solution en milieu aqueux pour usage.

Le produit peut être remis facilement et rapidement en solution en milieu aqueux, tel que de l'eau distillée, par exemple au moyen d'une seringue et sans nécessité, notamment d'étapes de filtration ultérieure comme pour le DAB présenté sous forme de poudre. En outre, le produit, même conservé à température ambiante, présente une stabilité suffisante pourvu qu'il soit conservé sous atmosphère d'azote ou d'un autre gaz inerte pour le préserver de tout risque d'oxydation au contact de l'air. Dans la pratique, conservée à 4°C, il présente une excellente stabilité.

La présente invention a également pour objet un procédé de lyophilisation du DAB.

D'autres caractéristiques et avantages de la présente invention apparaîtront à la lumière d'un exemple de réalisation du procédé selon l'invention.

La lyophilisation se fait à partir d'une solution de DAB dans une solution tampon Tris-Hcl 0,05 M à pH ajusté à 7,4 à raison de 0,5 mg/ml et sous atmosphère d'un gaz inerte, tel que l'argon ou l'azote.

Cette atmosphère inerte permet également d'éviter l'oxydation risquant de se produire au contact de l'air.

5 mg de DAB (Grade II de chez SIGMA) sont dilués dans 10 ml d'un tampon Tris-Hcl 0,05 M, pH 7,4. Le pH est ajusté à 7,4. Le mélange est lyophilisé selon un procédé classique de lyophilisation en présence d'argon ou d'azote.

Le DAB lyophilisé est conditionné dans des flacons prêts à l'emploi après reconstitution avec de l'eau distillée.

Les manipulations de ce produit toxique ne présentent plus les risques que l'on connaissait, et le produit stocké à 4°C est stable.

## Revendications

1. Technique enzymatique utilisant la révélation de la peroxydase avec comme chromogène le DAB (tétrahydrochlorure de 3,3' diaminobenzidine), caractérisée en ce qu'on fait usage du DAB conservé sous forme lyophilisée que l'on remet en solution en milieu aqueux.

2. Technique enzymatique selon la revendication 1, caractérisée en ce qu'il s'agit d'une technique immunologique, notamment de dosages immunologiques mettant en oeuvre des anticorps marqués à la peroxydase.

3. Technique enzymatique selon l'une des revendications 1 ou 2, caractérisée en ce qu'il s'agit d'une technique mettant en oeuvre des sondes d'acides nucléiques et un marquage à la peroxydase.

4. Trousse de réactifs pour la mise en oeuvre d'une technique selon l'une des revendications 1 à 3, notamment un kit de dosage et/ou diagnostique, caractérisée en ce qu'elle comporte du DAB présenté sous forme lyophilisée.

5. Procédé de lyophilisation du tétrahydrochlorure de 3,3' diaminobenzidine (DAB) pour la mise en oeuvre d'une technique selon l'une des revendications précédentes.

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| Y | EP-A-0 184 437  (EASTMAN KODAK CO.) <br> * Résumé; page 9, ligne 20 - page 11, ligne 20; page 13, ligne 32 - page 14, ligne 2 * <br> --- | 1-5 | C 12 Q    1/28 <br> G 01 N  33/535 |
| Y | EP-A-0 138 357  (INTEGRATED GENETICS INC.) <br> * Résumé; pages 19-20, exemple 5 * <br> --- | 1-5 | |
| Y | CHEMICAL ABSTRACTS, vol. 106, no. 21, 25 mai 1987, page 368, résumé no. 172336n, Columbus, Ohio, US; R. KOGA: "Comparison of various chromogens for horseradish peroxidase in enzyme immunoassay", & SEIRIGAKU KENKYUSHO GIJUTSUKA HOKOKU 1986, 1, 5-7 <br> * Résumé * <br> --- | 1-5 | |
| A | EP-A-0 205 844  (RICHARDSON-VICKS, INC.) <br> * Page 2, lignes 12-26; page 7, ligne 26 - page 9, ligne 7 * <br> ----- | 1,4 | DOMAINES TECHNIQUES RECHERCHES (Int. Cl.4) <br><br> C 12 Q <br> G 01 N |

Le présent rapport a été établi pour toutes les revendications

| Lieu de la recherche | Date d'achèvement de la recherche | Examinateur |
|---|---|---|
| LA HAYE | 17-08-1988 | HITCHEN C.E. |